(19) Europäisches Patentamt / European Patent Office / Office européen des brevets

(11) **EP 3 475 686 B1**

(12) **EUROPÄISCHE PATENTSCHRIFT**

(45) Veröffentlichungstag und Bekanntmachung des Hinweises auf die Patenterteilung:
**16.10.2024   Patentblatt 2024/42**

(21) Anmeldenummer: **17733352.3**

(22) Anmeldetag: **12.06.2017**

(51) Internationale Patentklassifikation (IPC):
**G01N 21/3504** *(2014.01)*   **G01N 33/22** *(2006.01)*
**G01N 21/03** *(2006.01)*   **G01N 21/94** *(2006.01)*
**G01N 21/85** *(2006.01)*

(52) Gemeinsame Patentklassifikation (CPC):
**G01N 21/3504; G01N 21/0317; G01N 21/94; G01N 33/225;** G01N 21/031; G01N 2021/3536; G01N 2021/354; G01N 2021/8557

(86) Internationale Anmeldenummer:
**PCT/EP2017/000682**

(87) Internationale Veröffentlichungsnummer:
**WO 2017/220189 (28.12.2017 Gazette 2017/52)**

(54) **VERFAHREN UND VORRICHTUNG ZUR ÜBERWACHUNG DER QUALITÄT VON GASFÖRMIGEN MEDIEN**

METHOD AND DEVICE FOR MONITORING THE QUALITY OF GASEOUS FLUIDS

MÉTHODE ET APPAREIL POUR SURVEILLANCE DES FLUIDS GAZEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.06.2016   DE 102016007825**

(43) Veröffentlichungstag der Anmeldung:
**01.05.2019   Patentblatt 2019/18**

(73) Patentinhaber: **Hydac Electronic GmbH**
**66128 Saarbrücken (DE)**

(72) Erfinder: **MANNEBACH, Horst**
**66127 Saarbrücken (DE)**

(74) Vertreter: **Bartels und Partner, Patentanwälte**
**Lange Strasse 51**
**70174 Stuttgart (DE)**

(56) Entgegenhaltungen:
WO-A2-2009/155459       DE-A1- 19 900 129
DE-C2- 19 900 129       GB-A- 2 466 181
US-A- 5 223 716       US-A1- 2011 005 302
US-A1- 2015 131 093       US-B2- 7 446 317

**Beschreibung**

Die Erfindung betrifft ein Verfahren und eine Vorrichtung zur Überwachung der Qualität bei von einer Tankstelle abgebbaren gasförmigen Medien, insbesondere Wasserstoff, mit den Schritten bzw. Merkmalen im Oberbegriff von Anspruch 1 bzw. 7.

Durch die WO 2013/139462 A1 ist eine Prüfvorrichtung zum Ermitteln der Partikelbelastung von unter einem hohen Druck stehendem Wasserstoff bekannt mit einem Gehäuse, das eine Probenkammer enthält, in der eine Filteraufnahme für einen Prüffilter vorgesehen ist, der bei einem Prüfvorgang von einer Probenmenge Wasserstoff durchströmbar und nach Ende des Prüfvorgangs aus der Probenkammer für die Auswertung der Anlagerung von Partikeln herausnehmbar ist, wobei ein einstellbares Drosselelement vorhanden ist, das eine unterschiedlich starke Drosselung der in die Probenkammer eintretenden Strömung ermöglicht.

Beim Einleiten eines Prüfvorgangs mit der bekannten Vorrichtung, bei dem die zunächst drucklose Probenkammer mit der Quelle für den unter hohem Druck stehenden Wasserstoff in Verbindung gebracht wird, beispielsweise einer Tanksäule für Wasserstoffbetankung, kann mittels des Drosselelements eine anfängliche Drosselung der Strömung derart eingestellt werden, dass der Prüffilter keinem schädlichen Druckschock ausgesetzt ist. Während dergestalt die Gefahr einer Beschädigung des Prüffilters durch einen anfänglichen Druckschock vermieden ist, kann für den weiteren eigentlichen Prüfvorgang die Drosselwirkung derart verringert werden, dass während eines nun stattfindenden Betankungsvorgangs ein Durchfluss durch den Prüffilter mit einer für das Prüfergebnis optimalen Durchflussrate stattfindet, die beispielsweise im Bereich von 60 g/sec liegen kann, also in einem Bereich, der für einen Betankungsvorgang eines Fahrzeugs geeignet ist.

Wie dargelegt, sind solche Vorrichtungen hauptsächlich für eine Anwendung bei Wasserstoff-Betankungssystemen oder Tankstellen nutzbar, also namentlich bei motorischen Anwendungen, bei denen Wasserstoff als gasförmiger Kraftstoff benutzt wird oder für die Versorgung von Brennstoffzellen mit Wasserstoff dient. Bei mit Wasserstoff betriebenen Verbrennungsmotoren ebenso wie bei Brennstoffzellen ist für den störungsfreien Betrieb ausschlaggebend, dass der Wasserstoff von partikulären Fremdstoffen völlig frei zu sein hat, was mit solchen Prüfvorrichtungen detektierbar ist.

Die bekannte Lösung eignet sich zwar sehr gut für das Detektieren von partikulären Verschmutzungen im Wasserstoffstrom; allein gasförmige Verunreinigungen des Wasserstoffs lassen sich dergestalt nicht feststellen, die ebenfalls einen schädigenden Einfluss auf einen Wasserstoffantrieb, insbesondere in Form einer Brennstoffzelle, haben können.

Die US 2015/0131093 A1 und die UK 2 466 181 A beschreiben jeweils ein Verfahren zur Überwachung der Qualität bei von einer Tankstelle abgebbaren gasförmigen Medien, insbesondere Wasserstoff, mittels einer Infrarot-Messeinrichtung, die in den Abgabeweg des jeweils gasförmigen Mediums zu einem Verbraucher geschaltet ist und die die Transmission von Infrarot-Strahlung bei unterschiedlichen Wellenlängen im Infrarot-Spektrum und Drücken misst, daraus die Konzentration von die Qualität beeinflussenden Verunreinigungen berechnet und zumindest bei Überschreiten vorgebbarer Qualitätsparameter dies zur Anzeige gebracht wird.

Weitere Verfahren zur Überwachung von gasförmigen Medien gehen aus der US 2011/0005302 A1, der US 5 223 716, der US 7 446 317 B2, der DE 199 00 129 A1 und der WO 2009/155459 A2 hervor.

Ausgehend von diesem Stand der Technik liegt daher der Erfindung die Aufgabe zugrunde, ein Verfahren sowie eine Vorrichtung zur Erhöhung der Genauigkeit der Überwachung der Qualität bei von einer Tankstelle abgebbaren gasförmigen Medien, insbesondere Wasserstoff, zur Verfügung zu stellen, mit dem sich auch gasförmige Verunreinigungen feststellen lassen.

Eine dahingehende Aufgabe löst ein Verfahren mit den Merkmalen des Patentanspruchs 1 in seiner Gesamtheit sowie eine Vorrichtung mit den Merkmalen des Patentanspruchs 7.

Gemäß dem Kennzeichen von Anspruch 1 ist vorgesehen, dass die Infrarot-Messeinrichtung zur Überwachung der Wasserstoffqualität an einer Wasserstoff-Tankstelle eingesetzt und zur Online-Messung von Gas-Verunreinigungen in den Abgabeweg ausgehend von der Tankstelle geschaltet wird; dass das abgegebene Medium Wasserstoff ist; dass die Probenanalyse mittels der Infrarot-Messeinrichtung bei sehr hohen Drücken von >500 bar vorgenommen wird; dass die Infrarot-Messeinrichtung derart in den Abgabeweg geschaltet ist, dass in einem Bypass zu diesem Abgabeweg mittels einer Ventileinrichtung in Hintereinanderabfolge Proben entnommen und auf Verunreinigungen hin überprüft werden, ohne dass dabei die jeweilige Abgabe von Wasserstoff von der Tankstelle zum angeschlossenen Verbraucher hin unterbrochen wird; und dass eine hochdruckfest ausgebildete Infrarot-Küvette mit einem druckfesten Rohrstück verwendet wird, wobei sich im Inneren des Rohrstücks ein Infrarot-Hohlleiter befindet, der als galvanisch vergoldetes Innenrohr ausgebildet in das Rohrstück eingeschoben und dort fixiert ist.

Bei dem erfindungsgemäßen Verfahren kommt eine Infrarot(IR)-Messeinrichtung zum Einsatz, die in den Abgabeweg des jeweils gasförmigen Mediums, insbesondere von Wasserstoff, ausgehend von der Tankstelle zu einem Verbraucher geschaltet ist und die die Transmission von Infrarot-Strahlung, respektive Infrarot-Licht bei unterschiedlichen Wellenlängen und Drücken misst, daraus die Konzentration von die Qualität beeinflussenden Verunreinigungen berechnet und zumindest bei Überschreiten vorgebbarer Qualitätsparameter dies zur Anzeige gebracht wird.

Gasförmige Verunreinigungen im Wasserstoff, wie sie regelmäßig aus der vorausgehenden Aufbereitung (Reformierung) oder aus Kompressorschäden entstehen, verursachen unter anderem Schäden an den empfindlichen Brennstoffzellen eines motorischen Antriebs. Daher geben Normen, wie die SAE J2719 oder die ISO/DIS 14647-2, Reinheitsanforderungen für den Wasserstoff an Wasserstoff-Tankstellen vor. Regelmäßige Verunreinigungen des Wasserstoffs sind neben Wasser auch Kohlenwasserstoffe. Ferner treten als gasförmige Verschmutzungen Kohlenmonoxide und Kohlendioxide auf. Des Weiteren sind im Wasserstoffgas als Verunreinigungen Formaldehyde vorhanden sowie Ameisensäure und Ammoniak, jeweils auch in Gasform. Weitere Verunreinigungen können aus den Gasen Sauerstoff, Helium, Stickstoff, Argon und Halogenen gebildet sein sowie Schwefelgas. Alle vorstehend genannten gasförmigen Verunreinigungen des Wasserstoffs einschließlich des Wassers lassen sich im Labor feststellen, wobei aufgrund der Vielzahl der Verbindungen und der niedrigen Grenzwerte eine entsprechende Labor-Analyse bereits eine große Herausforderung darstellt und den Einsatz mehrerer Messmethoden, wie unterschiedliche Methoden der Gaschromatographie, sowie der Ionenchromatographie erfordert. Eine direkte Übertragung dieser Labor-Messmethoden auf eine Sensor-Vorrichtung zur Online-Messung der Wasserstoffqualität an einer Tankstelle ist dabei nicht möglich. Eine Analysemethode mit der größten Abdeckung der besonders kritischen gasförmigen Verunreinigungen im Sinne der erfindungsgemäßen Lösung ist die Infrarot-Spektroskopie. Bei der Infrarot-Spektroskopie nutzt man aus, dass die zu detektierenden Stoffe Licht im Bereich des Infrarot-Spektrums absorbieren. Die Absorption erfolgt dabei jeweils bei stoffspezifischen Wellenlängen. Dabei sind nicht alle Substanzen infrarotaktiv, d.h. nicht alle absorbieren infrarotes Licht; vielmehr müssen infrarotaktive Substanzen im Allgemeinen ein Dipolmoment besitzen. Symmetrische Moleküle, wie der vorstehend genannte Sauerstoff, Helium, Stickstoff, Argon sowie Halogene, lassen sich wegen fehlendem Dipolmoment, mit der erfindungsgemäßen Messmethode nicht erfassen. Alle anderen genannten gasförmigen Verunreinigungen im Wasserstoff lassen sich jedoch mit der Infrarot-Spektroskopie unter Einsatz der erfindungsgemäßen Infrarot-Messeinrichtung vor Ort und online an einer Wasserstofftankstelle während des Betankungsvorgangs durchführen.

Als besonders vorteilhaft hat es sich dabei auch erwiesen, das erfindungsgemäße Verfahren unter Einsatz der Infrarot-Messeinrichtung trotz der hohen Drücke einer Wasserstoffabgabe an der Tankstelle online durchführen zu können, so dass der Betankungsvorgang durch die Qualitätsmessung nicht beeinträchtigt wird. Dergestalt kann an der Tankstelle online ein Messprotokoll erstellt werden, das man dem Tankstellennutzer als Nachweis für die Reinheit des abgegebenen Wasserstoffs an die Hand gibt, um diesem die Gewährleistung zu geben, dass der Brennstoffzellenbetrieb seines Fahrzeugs mit dem getankten Wasserstoff weiter ungestört verläuft.

Zum Durchführen des dahingehenden Infrarot-Messverfahrens dient eine erfindungsgemäße Vorrichtung mit einer hochdruckfesten Infrarot-Küvette, bestehend aus einem druckfesten Rohrstück, in das ein galvanisch vergoldetes Innenrohr als Infrarot-Rohrleiter eingeschoben und dort fixiert wird. Absperrbare Anschlüsse für Probeneinlass und -auslass dienen der Wasserstoffzufuhr oder -abfuhr in bzw. aus dem druckfesten Rohrstück für einen Infrarot-Messvorgang mittels eines geeigneten Emitters sowie eines geeigneten Detektors. Die ermittelten Werte lassen sich dann beispielsweise für das Erstellen eines Messprotokolls oder für die Ausgabe eines Alarms an eine Steuer- und Recheneinheit anschließen, die eine Signalverarbeitung auch unter Einbezug von Druck- und/oder Temperaturwerten des Wasserstoffs im Online-Betrieb durchführen kann.

Weitere vorteilhafte Ausführungen der erfindungsgemäßen Lösung sind Gegenstand der sonstigen Unteransprüche.

Eine erfindungsgemäße Messvorrichtung weist die Merkmale des Anspruchs 7 auf. Vorteilhafte Ausführungsformen der Messvorrichtung gehen aus den von Anspruch 7 abhängigen Unteransprüchen hervor.

Im Folgenden wird das erfindungsgemäße Verfahren anhand einer erfindungsgemäßen Vorrichtung zum Durchführen dieses Verfahrens näher erläutert. Dabei zeigen in prinzipieller und nicht maßstäblicher Darstellung die

Fig. 1 und 2    zwei verschiedene Arten von erfindungsgemäßen Messvorrichtungen;

Fig. 3    eine ergänzte Vorrichtungslösung zu der Ausführungsform nach der Fig. 1; und

Fig. 4 bis 6    verschiedene Infrarot-Spektren für Kohlenmonoxid, Kohlendioxid und Ethan, wobei über der Abszisse mit den Wellenzahlen in 1/cm die Ordinate mit der jeweiligen Absorbanz A aufgetragen ist.

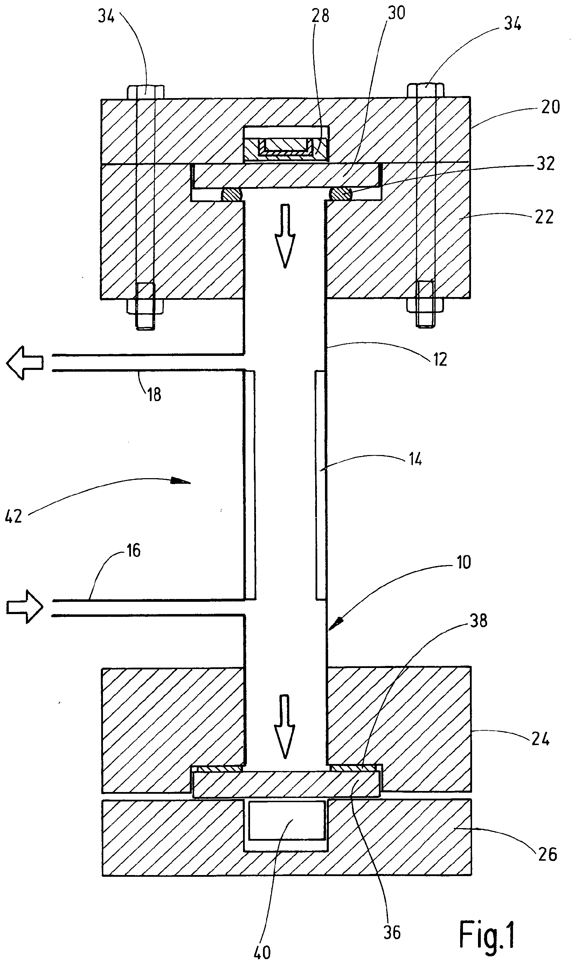

Die erfindungsgemäße Vorrichtung nach der Fig. 1 zeigt eine hochdruckfest ausgebildete Infrarot-Küvette 10 mit einem druckfesten Rohrstück 12 auf.

Im Inneren des Rohrstücks 12 befindet sich ein Infrarot-Hohlleiter 14, der als galvanisch vergoldetes Innenrohr ausgebildet in das Rohrstück 12 eingeschoben und dort gemäß der Darstellung nach der Fig. 1 fixiert ist. Unterhalb des Hohlleiters 14 mündet ein rohrförmiger Anschluss 16 medienführend in das Rohrstück 12 ein, ebenso ein weiterer rohrförmiger Anschluss 18 oberhalb des Hohlleiters 14. Der Anschluss 16 dient, wie die Pfeildarstellung zeigt, der Zufuhr eines gasförmigen Mediums und der Anschluss 18 der Abfuhr der dahingehend in das Rohrstück 12 eingeleiteten Gasmenge. Erfindungsgemäß wird zur Untersuchung Wasserstoff unter sehr hohem Druck, beispielsweise im Bereich von 70 MPa (700 bar), der Infrarot-Küvette 10, ausgehend von einer Wasserstoff-Tankstelle (nicht dargestellt) als Probe zugeführt.

An die gegenüberliegend freien Enden des Rohrstücks 12 grenzen paarweise einander zugeordnete Flanschteile 20, 22 und 24, 26 an. Das in der Fig. 1 zuoberst angeordnete erste Flanschteil 20 weist eine Mittenausnehmung für die Aufnahme eines Hochleistungs-Infrarot-Emitters 28 auf. Darunterliegend und in einer Ausnehmung des zweiten Flanschteils 22 aufgenommen befindet sich ein infrarotdurchlässiges, hochdruckfestes Fenster 30, das über eine stirnseitig angeordnete Ringdichtung 32 verfügt, die das Innere des Rohrstücks 12 gegenüber der Umgebung abdichtet. Wie in der Fig. 1 ferner angedeutet, sind die beiden Flanschteile 20 und 22 über eine Schraubverbindung 34 unter Einbeziehung der Ringdichtung 32 druckdicht miteinander verbunden. Als Material für das Fenster 30 dient insbesondere Silizium und Germanium, das Infrarot-Strahlung in einem vorgebbaren Wellenbereich durchlässt.

Das das Rohrstück 12 nach unten abschließende Flanschteil 24 weist wiederum eine Mittenausnehmmung auf, in die ein weiteres Infrarot durchlässiges Fenster 36 eingesetzt ist, das über eine Lötverbindung 38, die stirnseitig auf der Oberseite des Fensters 36 angeordnet ist, die Abdichtung des Inneren des Rohrstücks 12 gegenüber der Umgebung vornimmt. In das darunterliegende Flanschteil 26 ist dann in eine Mittenausnehmung desselben ein Infrarot-Detektor 40 eingesetzt, der der Auswertung des empfangenen Infrarot-Spektrums dient. Mithin befinden sich im drucklosen Bereich hinter dem jeweiligen Fenster 30, 36 der Infrarot-Emitter 28 bzw. der Infrarot-Detektor 40.

In der hochdruckfesten Küvette 10 steigt die Volumenkonzentration der gasförmigen Verunreinigungen proportional mit dem Druck an, so dass z.B. bei einem Eintragsdruck des Wasserstoffs von 50 MPa (500 bar) die Infrarot-Absorption in der Messzelle das 500-fache beträgt gegenüber einem Eintragsdruck von 0,1 MPa (1 bar).

Diese Vervielfachung des Messeffekts ermöglicht den Einsatz der aufgezeigten robusten und kostengünstig zu verwirklichenden IR-Infrarot-Messtechnik.

Die in Fig. 1 gezeigte Messanordnung oder Messvorrichtung bildet mithin die für das Verfahren als Ganzes vorgesehene Infrarot-Messeinrichtung 42 aus.

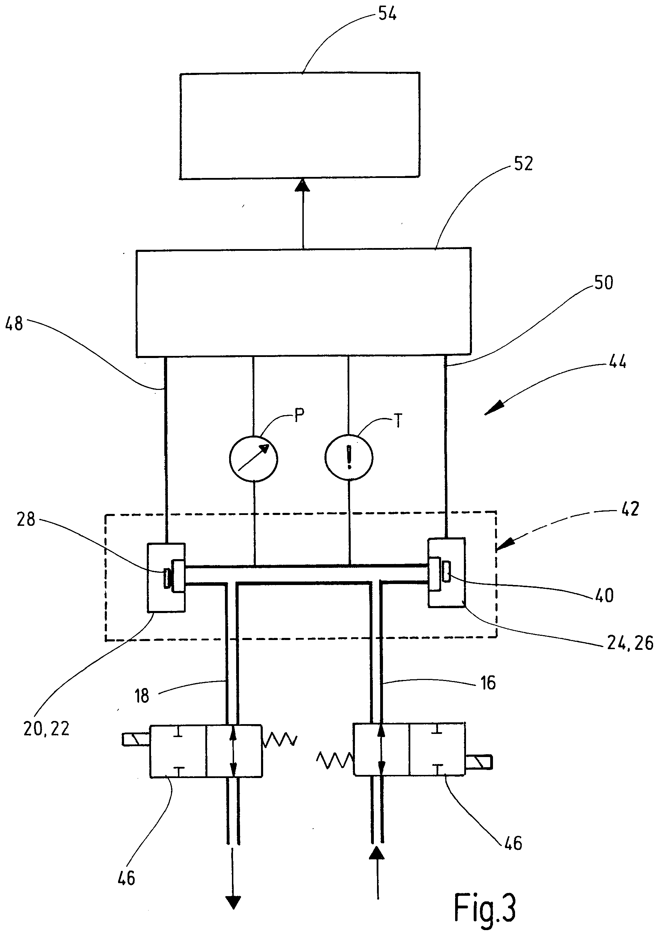

Wie nun beispielhaft die Darstellung nach der Fig. 3 zeigt, ist die Infrarot-Messeinrichtung 42 nach der Fig. 1 Bestandteil einer Gesamt-Messeinrichtung 44, die man zur Online-Messung von Gas-Verunreinigungen an eine Abgabe-Tankstelle, beispielsweise für Wasserstoff, mit geringem Montageaufwand anschließen kann. Zum Absperren des Zu- und Abflusses von Wasserstoffgas sind in die Anschlüsse 16, 18 elektromagnetisch betätigbare 2/2-Wege-Ventile 46 geschaltet. Mithin lässt sich über die Messeinrichtung 44 also Wasserstoffgas online aus der Tank-Abgabeleitung (nicht dargestellt) entnehmen und für einen Messvorgang nach Schließen der Wegeventile 46 in der Infrarot-Küvette 10 für einen Auswertevorgang aufnehmen.

Dabei sind sowohl der Infrarot-Emitter 28 als auch der Infrarot-Detektor 40 über entsprechende elektrische Verbindungsleitungen 48 bzw. 50 an eine signalverarbeitende Elektronik angeschlossen, die in der Fig. 3 in der Art einer Blackbox als Steuer- und Recheneinheit 52 wiedergegeben ist. In die dahingehende Signalverarbeitung kann über entsprechende Sensoren P, T auch der jeweilige Druckwert bzw. der Temperaturwert in der Messstrecke, gebildet durch das Innere des Rohrstücks 12, erfasst und für die Auswertung der ermittelten optischen Ergebnisse mit eingesetzt werden. Ausgangsseitig an die signalverarbeitende Steuer- und Recheneinheit 52 kann wiederum ein Ausgabegerät 54 angeschlossen sein, das beispielsweise ein Messprotokoll erstellt über die Qualität (Verunreinigungsgrad und Art der Verunreinigungen) des mittels der Tankstelle abgegebenen Wasserstoffs und vorzugsweise im Bedarfsfall bei Überschreiten vorgebbarer Qualitätsparameter des Gases einen Alarm auslöst.

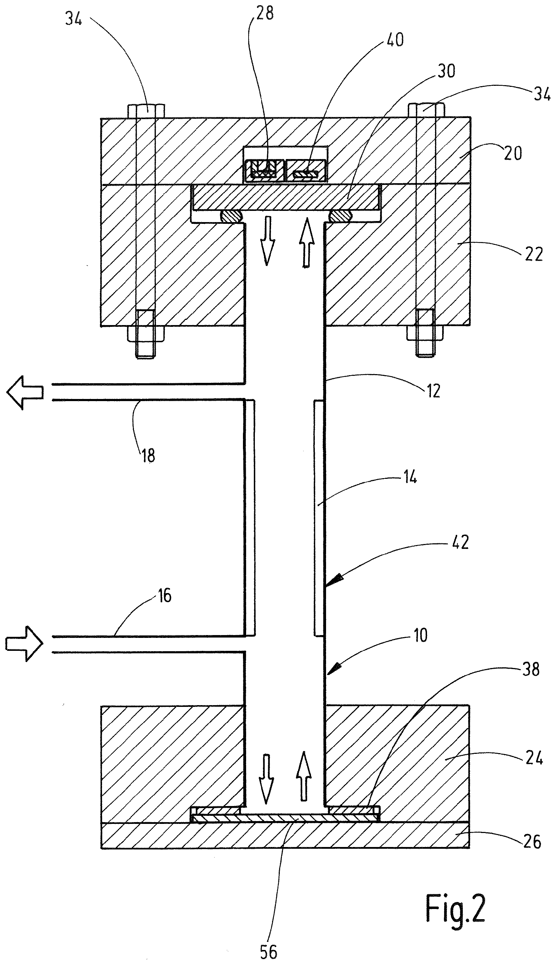

Die Ausführungsform nach der Fig. 2 entspricht weitestgehend der Infrarot-Messeinrichtung 42 gemäß der Ausführungsform nach der Fig. 1 mit der Maßgabe, dass in dem oberen Flanschteil 20 in dessen Mittenausnehmung sowohl der Infrarot-Emitter 28 als auch der Infrarot-Detektor 40 nebeneinander untergebracht sind. Unterschiedlich ist weiter, dass wiederum über eine Lötverbindung 38 nunmehr ein Infrarotstrahlen reflektierender Spiegel 56 am unteren Ende des Rohrstücks 12 im dritten Flanschteil 24 aufgenommen ist. Über eine nicht näher dargestellte Schraubverbindung, vergleichbar der Schraubverbindung 34, sind dann auch die unteren beiden Flanschteile 24 und 26 derart fest miteinander verbunden, dass über die untere Lötverbindung 38 ein druckdichter Abschluss des Inneren des Rohrstücks 12 gegenüber der Umgebung erreicht ist. Wie die Doppelpfeil-Darstellung im Inneren des Rohrstücks 12 zeigt, ist aufgrund des Spiegels 56 die Wegmessstrecke zwischen Emitter 28 und Detektor 40 gegenüber der Lösung nach der Fig. 1 verdoppelt, was insoweit die Messgenauigkeit der Messeinrichtung 42 gemäß der Ausgestaltung nach der Fig. 2 entsprechend erhöht.

Die vorgestellte erfindungsgemäße Lösung dient dem Durchführen eines Verfahrens zur Überwachung der Wasserstoffqualität an einer nicht näher dargestellten Wasserstofftankstelle mit einer Gesamt-Messeinrichtung 44 gemäß dem Ausführungsbeispiel nach der Fig. 3, bestehend im Wesentlichen aus der bereits vorgestellten hochdruckfesten Infrarot-Küvette 10, einem Infrarot-Emitter 28, einem Infrarot-Detektor 40 sowie zumindest einem Drucksensor P und einer Steuer- und Recheneinheit 52, wobei die Messeinrichtung 44 als Ganzes in die Wasserstofftankstelle integriert ist und im Bedarfsfall periodisch mittels den Ventilen 46 für Messvorgänge mit Wasserstoff gefüllt und wieder entleert werden kann. Mit der dahingehenden Infrarot-Messeinrichtung 42, 44 wird dann die Transmission von infraroter Strahlung oder infrarotem Licht bei unterschiedlichen Wellenlängen und Drücken gemessen und daraus die Konzentration von insbesondere gasförmigen Verunreinigungen im Wasserstoff berechnet. Geben die ermittelten Messwerte Grund zur Besorgnis, kann bei Überschreiten von vorgebbaren Grenzwerten das an die Steuer- und Recheneinheit 52 in Form einer elektronischen Signalverarbeitung angeschlossene Ausgabegerät 54 einen Alarm ausgeben.

Vorzugsweise führt dabei die Steuer- und Recheneinheit 52 Transmissionsmessungen bei unterschiedlichen Dichten durch, speichert diese zwischen und subtrahiert diese dann wieder voneinander. Wie bereits dargelegt, kann dabei zur Bestimmung der Dichte eine Druckmessung über einen Drucksensor P erfolgen, wobei die Druckmessung um eine Temperaturmessung mittels eines Temperatursensors T ergänzt sein kann.

Als besonders vorteilhaft für das Durchführen des erfindungsgemäßen Verfahrens hat es sich gezeigt, als Infrarot-Emitter 28, vorzugsweise einen Hotplate-Emitter einzusetzen, der einen breitbandigen Infrarotstrahl abgibt. Des Weiteren ist es vorteilhaft, wenn der Infrarot-Detektor 40 ein mehrkanaliger Detektor mit Filtern im Bereich zwischen 1000 und 4000 Wellenzahlen ist. Vorteilhafterweise ist dabei der Infrarot-Detektor 40 ein Spektrometer. Sofern sich gemäß der Darstellung nach der Fig. 2 Emitter 28 und Detektor 40 auf der gleichen Seite des Rohrstücks 12, also im oberen Flanschteil 20, befinden, benötigt man nur eine gemeinsame Elektronik für die dahingehenden Bauteile 28 und 40.

Was im Folgenden noch näher erläutert werden wird, zeigt die Verteilung der detektierten infrarotaktiven Banden der einzelnen Verschmutzungen, aufgetragen über der Wellenzahl, die Art der jeweiligen Verunreinigung auf. So findet man bei Wellenzahlen um 1000 Ammoniakgas als Verunreinigung und im Bereich von einer 2000er Wellenzahl die Verunreinigungen Ameisensäure, Wasser, Kohlenmonoxid und Kohlendioxid. Bei Wellenzahlen um 3000 Formaldehyd und Kohlenwasserstoffe und bei Wellenzahlen knapp vor 4000 wiederum Wasser. Wie also bereits dargelegt, liegt der relevante Messbereich zwischen 1000 und 4000 Wellenzahlen, d.h. zwischen 2,5 $\mu$m und 10 $\mu$m Wellenlänge der infraroten Strahlung. Aus der Lage der Absorptionsbanden kann man also, wie dargelegt, eine Substanz an sich identifizieren und aus der Abschwächung der infraroten Strahlung beim Durchgang durch die Wasserstoffprobe ergibt sich die Konzentration aus dem Lambert-Beer'schen Gesetz wie folgt:

$$A = \lg(I_0 / I_1) = \lg(1/T) = \varepsilon(\lambda) \cdot c \cdot d$$

mit den Begriffen:

A: Absorbanz
$I_0$: Intensität des eingestrahlten Lichts [W/m$^2$]
$I_1$: Intensität des abgeschwächten Lichts [W/m$^2$]

T = $I_1/I_0$: Transmissionsgrad
$\varepsilon(\lambda)$: molarer (dekadischer) Absorptionskoeffizient [m$^2$/mol]
c: Konzentration [mol/L]
d: Weglänge des Lichtstrahls durch die Probe [m]

Die Wellenlängenabhängigkeit der Absorption findet sich also im Lambert-Beer'schen Gesetz in der Wellenlängenabhängigkeit des molaren Absorptionskoeffizienten wieder.

Aus dem Lambert-Beer'schen Gesetz ist der Standardansatz zur Messung kleiner Konzentrationen ersichtlich. Bei kleinen Konzentrationen wird die Weglänge d des Infrarotlichtstrahls durch die Probe vergrößert. Dies kann durch eine Verlängerung der Messzelle in Form der Küvette 10 oder durch ein mehrfaches Durchstrahlen der Küvette 10 über eine Spiegelvorrichtung mit dem Spiegel 56 gemäß der Darstellung nach der Fig. 2 geschehen. Für die hier angesprochene Anwendung ist der Verlängerung der Weglänge d jedoch eine praktische Grenze gesetzt und es hat sich als vorteilhaft erwiesen, das als Messstrecke ausgebildete Rohrstück 12 nach den Fig. 1 und 2 vorzugsweise in einer Länge von 500 mm auszubilden, wobei auch Längen von 100 mm bis 1500 mm durchaus möglich sind. Ferner beträgt der Durchmesser des Rohrstücks 10, vorzugsweise 3 bis 15 mm, besonders bevorzugt 5 mm.

Des Weiteren muss die Konzentration c betrachtet werden, die noch eine andere beeinflussbare Größe auf der rechten Seite der vorstehenden Lambert-Beer'schen Gleichung bildet. c bezeichnet, wie dargelegt, die Konzentration des zu detektierenden Stoffes in der Infrarot-Küvette 10 in mol/L. Diese Größe c lässt sich ohne weiteres durch eine Druckerhöhung signifikant vergrößern und dies ist im Falle der Wasserstofftankstelle besonders vorteilhaft, da hier das zu analysierende Gas bereits unter sehr hohem Druck vorliegt, beispielsweise in der Größenordnung von 70 bis 80 MPa (700 bis 800 bar).

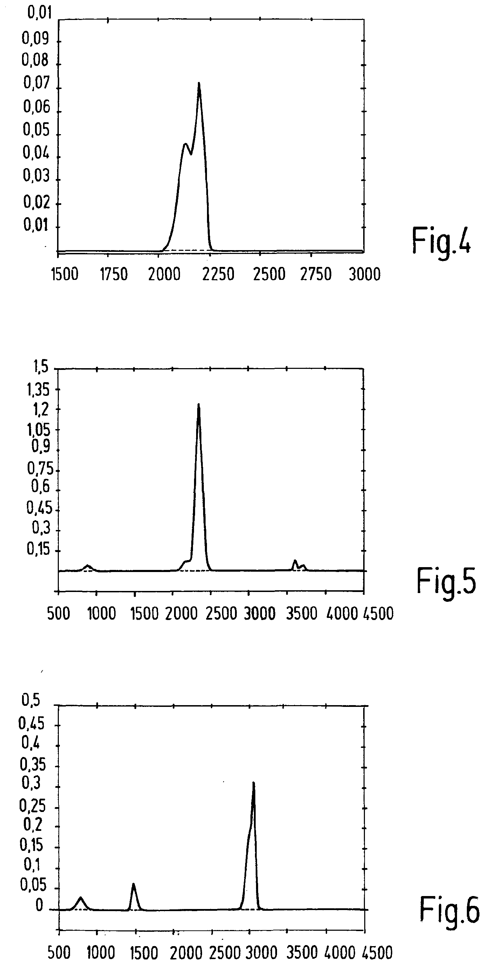

Die Infrarotspektren nach den Fig. 4 bis 6 machen deutlich, dass bei den sehr hohen Drücken von 70 MPa (700 bar) sich sofort ein signifikantes Messbild (durchgezogen gezeichnete Linien) ergibt, was bei 0,1 MPa (1 bar) nicht gegeben ist (gestrichelt wiedergegebene Messlinien).

Die in Fig. 4 gemessene Absorbanz des IR-Spektrums von etwa 0,06 bei CO-Gas, bei einer Konzentration von 100 ppm, einem Druck von 0,1 MPa (1 bar) absolut und einer Weglänge von 10m, bedeutet, dass bei 2200 Wellenzahlen noch etwa 87 % der Intensität transmittiert werden. Der in der Norm SAE J2719 vorgegebene Grenzwert für Kohlenmonoxid beträgt jedoch nur 0,2 ppm, d.h. ein Fünfhundertstel der Konzentration der in Fig. 4 gezeigten Messung. Die Absorbanz beträgt bei dieser Konzentration nun 0,00012 und die Transmission 99,97 %. Diese geringe Transmissionsänderung wäre mit Laborgeräten nur äußerst schwer nachweisbar. Erhöht man nun den Druck auf 70 MPa (700 bar) gemäß der durchgezogen gezeichneten Messliniendarstellung nach der Fig. 4, so steigt auch die Absorbanz um den Faktor 700 an. Bei einer Reduktion der Weglänge d von ca. 10 m auf 50 cm gemäß der Längenausbildung des Rohrstücks 12 für die Infrarot-Küvette 10 verliert man bei der Absorbanz dann wieder einen Faktor 20 und erreicht einen Wert von 0,0042. Dieser Wert entspricht jedoch wiederum einer Transmission von 99,03 %. Diese Abschwächung von fast 1 % ist auch mit optischen Standardkomponenten gut nachweisbar. Entsprechende Abschätzungen für Wasser, Kohlenwasserstoffe, Kohlendioxid (s. Fig. 5), Formaldehyd, Ameisensäure, Ammoniak und Ethan (s. Fig. 6) bestätigen die obige Betrachtung und zeigen auf, dass der entscheidende Faktor die Erhöhung der Konzentration innerhalb der hochdruckfesten IR-Küvette 10, hervorgerufen durch den Abgabedruck des Wasserstoffes an der Tankstelle, ist.

Wie die Fig. 4 bis 6 letztendlich zeigen, lassen sich mit der vorstehend beschriebenen erfindungsgemäßen Vorrichtung nach den Fig. 1 bis 3 im Wasserstoffgas bei hohen Drücken ohne weiteres gasförmige Verunreinigungen, wie Kohlenmonoxid (Fig. 4), Kohlendioxid (Fig. 5) oder Ethan (Fig. 6) verlässlich nachweisen und bei Überschreiten vorgebbarer Grenzwerte lässt sich der Wasserstofftankvorgang vorteilhafter Weise unterbrechen.

## Patentansprüche

1. Verfahren zur Überwachung der Qualität bei von einer Tankstelle abgebbaren gasförmigen Medien mittels einer Infrarot-Messeinrichtung (42), die in den Abgabeweg des jeweils gasförmigen Mediums zu einem Verbraucher geschaltet ist und die die Transmission von Infrarot-Strahlung bei unterschiedlichen Wellenlängen im Infrarot-Spektrum und Drücken misst, daraus die Konzentration von die Qualität beeinflussenden Verunreinigungen berechnet und zumindest bei Überschreiten vorgebbarer Qualitätsparameter dies zur Anzeige gebracht wird, **dadurch gekennzeichnet,**

    **dass** die Infrarot-Messeinrichtung (42) zur Überwachung der Wasserstoffqualität an einer Wasserstoff-Tankstelle eingesetzt und zur Online-Messung von Gas-Verunreinigungen in den Abgabeweg ausgehend von der Tankstelle geschaltet wird;
    **dass** das abgegebene Medium Wasserstoff ist;
    **dass** die Probenanalyse mittels der Infrarot-Messeinrichtung (42) bei sehr hohen Drücken von > 50 MPa vorgenommen wird;
    **dass** die Infrarot-Messeinrichtung (42) derart in den Abgabeweg geschaltet ist, dass in einem Bypass zu diesem Abgabeweg mittels einer Ventileinrichtung (46) in Hintereinanderabfolge Proben entnommen und auf Verunreinigungen hin überprüft werden, ohne dass dabei die jeweilige Abgabe von Wasserstoff von der Tankstelle zum angeschlossenen Verbraucher hin unterbrochen wird; und
    **dass** die Infrarot-Messeinrichtung (42) eine hochdruckfest ausgebildete Infrarot-Küvette (10) mit einem druckfesten Rohrstück (12) umfasst, wobei sich im Inneren des Rohrstücks (12) ein Infrarot-Hohlleiter (14) befindet, der als galvanisch vergoldetes Innenrohr ausgebildet in das Rohrstück (12) eingeschoben und dort fixiert ist.

2. Verfahren nach Anspruch 1, **dadurch gekennzeichnet, dass** mittels der Infrarot-Messeinrichtung (42) Verunreinigungen erfasst werden, die aufgrund eines Dipolmomentes infrarotaktiv sind.

3. Verfahren nach Anspruch 1 oder 2, **dadurch gekennzeichnet, dass** die Infrarot-Messeinrichtung (42) unter Einsatz der Infrarot-Spektroskopie aus der Lage von Absorptionsbanden einer Probe die Art der Verunreinigung ermittelt und aus der Abschwächung der Infrarot-Strahlung beim Durchgang derselben durch die Probe die Konzentration der jeweiligen Verunreinigungen ermittelt.

4. Verfahren nach einem der vorstehenden Ansprüche, **dadurch gekennzeichnet, dass** zur Ermittlung der Konzentration der jeweiligen Verunreinigung in der Probe das Lambert-Beer'sche Gesetz zur Anwendung kommt.

5. Verfahren nach einem der vorstehenden Ansprüche, **dadurch gekennzeichnet, dass** die Probenanalyse mittels der Infrarot-Messeinrichtung (42) bei sehr hohen Drücken von > 70 MPa vorgenommen wird.

6. Verfahren nach einem der vorstehenden Ansprüche, **dadurch gekennzeichnet, dass** eine Steuer- und Recheneinheit (52) der Infrarot-Messeinrichtung (42) Transmissionsmessungen an der jeweiligen Probe bei unterschiedlichen Dichten zwischenspeichert und für Bestimmung der Konzentration voneinander subtrahiert und dass zur Bestimmung der Probendichte eine Druckmessung und im Bedarfsfall zusätzlich eine Temperaturmessung der Probe vorgenommen wird.

7. Messvorrichtung (44) zur Überwachung der Qualität bei von einer Tankstelle abgebbaren gasförmigen Medien, insbesondere Wasserstoff,

    wobei die Messvorrichtung (44) eine Infrarot-Messeinrichtung (42) mit einer Infrarot-Küvette (10), einem Infrarot-Emitter (28) und einem Infrarot-Detektor (40) umfasst, welche in den Abgabeweg des jeweils gasförmigen Mediums zu einem Verbraucher schaltbar ist, wobei die Messvorrichtung (44) ferner die folgenden Komponenten umfasst:

- einen Drucksensor (P);
- eine Steuer- und Recheneinheit (52);
- eine Ventileinrichtung (46); und
- Anschlüsse (16, 18) zum Zuführen und Ausleiten, mittels der Ventileinrichtung (46), einer Probe des gasförmigen Mediums in die und aus der Infrarot-Küvette (10);

wobei ausgangsseitig an die signalverarbeitende Steuer- und Recheneinheit (52) ein Ausgabegerät (54) angeschlossen ist, und

wobei die Steuer- und Recheneinheit (52) dazu ausgerichtet ist, die Probenanalyse durchzuführen und dabei die Transmission von Infrarot-Strahlung durch die sich in der Infrarot-Küvette (10) befindliche Probe bei unterschiedlichen Wellenlängen im Infrarot-Spektrum und Drücken zu messen, daraus die Konzentration von die Qualität beeinflussenden Verunreinigungen zu berechnen und zumindest bei Überschreiten vorgebbarer Qualitätsparameter dies zur Anzeige zu bringen;
**dadurch gekennzeichnet,**
**dass** die Messvorrichtung (44) zur Überwachung der Wasserstoffqualität an eine Wasserstoff-Tankstelle anschließbar oder in diese integriert ist;
**dass** die Infrarot-Messeinrichtung (42) zur Online-Messung von Gas-Verunreinigungen in den Abgabeweg ausgehend von der Tankstelle geschaltet ist;
**dass** das abgegebene Medium Wasserstoff ist;
**dass** die Infrarot-Küvette (10) im Bedarfsfall periodisch mittels der Ventileinrichtung (46) für Messvorgänge mit einer Probe des Wasserstoffs füllbar und wieder entleerbar ist;
**dass** die Probenanalyse mittels der Infrarot-Messeinrichtung (42) bei sehr hohen Drücken von > 50 MPa vorgenommen wird;
**dass** die Infrarot-Messeinrichtung (42) derart in den Abgabeweg schaltbar ist, dass in einem Bypass zu diesem Abgabeweg mittels der Ventileinrichtung (46) in Hintereinanderabfolge Proben entnehmbar und auf Verunreinigungen hin überprüfbar sind,
ohne dass dabei die jeweilige Abgabe von Wasserstoff von der Tankstelle zum angeschlossenen Verbraucher hin unterbrochen wird; und
**dass** die Infrarot-Küvette (10) hochdruckfest ausgebildet ist und mit einem druckfesten Rohrstück (12) versehen ist, wobei sich im Inneren des Rohrstücks (12) ein Infrarot-Hohlleiter (14) befindet, der als galvanisch vergoldetes Innenrohr ausgebildet in das Rohrstück (12) eingeschoben und dort fixiert ist.

8. Messvorrichtung nach Anspruch 7,
**dadurch gekennzeichnet, dass** das druckfeste Rohrstück (12) an zumindest einem Rohrende ein Flanschteil (22; 24) umfasst, das ein druckfestes, Infrarot-Strahlung durchlässiges Fenster (30) und/oder einen Infrarot-Strahlung reflektierenden Spiegel (56) aufweist.

**Claims**

1. Method for monitoring the quality of gaseous media which can be dispensed by a filling station, by means of an infrared measuring device (42), which is connected in the dispensing path of the gaseous medium to a consumer in each case and which measures the transmission of infrared radiation at different wavelengths in the infrared spectrum and different pressures, calculates therefrom the concentration of contaminants which influence the quality and displays this at least when predefinable quality parameters are exceeded, **characterised in that**

the infrared measuring device (42) is used for monitoring the hydrogen quality at a hydrogen filling station and is connected in the dispensing path, starting from the filling station, for online measurement of gas contaminants; **in that** the medium dispensed is hydrogen; **in that** the sample analysis is carried out by means of the infrared measuring device (42) at very high pressures of >50 MPa;
**in that** the infrared measuring device (42) is connected in the dispensing path in such a manner that samples are taken in succession in a bypass to this dispensing path by means of a valve device (46) and examined for contaminants without the respective dispensing of hydrogen from the filling station to the connected consumer being interrupted; and **in that** the infrared measuring device (42) comprises a high-pressure-resistant infrared cuvette (10) with a pressure-resistant pipe section (12), wherein an infrared hollow conductor (14) is located inside the pipe section (12), which hollow conductor, configured as a galvanically gold-plated inner tube, is pushed into the pipe section (12) and fixed therein.

2. Method according to claim 1, **characterised in that**, contaminants which are infrared-active due to a dipole moment are detected by means of the infrared measuring device (42).

3. Method according to claim 1 or 2, **characterised in that**, the infrared measuring device (42) uses infrared spectroscopy to determine the type of contaminant from the position of absorption bands of a sample and determines the concentration of the respective contaminants from the attenuation of the infrared radiation as it passes through the sample.

4. Method according to one of the preceding claims, **characterised in that**, the Beer-Lambert law is applied to determine the concentration of the respective contaminant in the sample.

5. Method according to one of the preceding claims, **characterised in that**, the sample analysis is carried out by means of the infrared measuring device (42) at very high pressures of >70 MPa.

6. Method according to one of the preceding claims, **characterised in that**, a control and computing unit (52) of the infrared measuring device (42) temporarily stores transmission measurements on the respective sample at different densities and subtracts them from one another to determine the concentration and **in that** a pressure measurement and, if necessary, additionally a temperature measurement of the sample is carried out to determine the sample density.

7. Measuring apparatus (44) for monitoring the quality of gaseous media which can be dispensed by a filling station, in particular hydrogen, wherein the measuring apparatus (44) comprises an infrared measuring device (42) with an infrared cuvette (10), an infrared emitter (28) and an infrared detector (40), which can be connected in the dispensing path of the gaseous medium to a consumer in each case, wherein the measuring apparatus (44) further comprises the following components:

    - a pressure sensor (P);
    - a control and computing unit (52);
    - a valve device (46); and
    - connections (16, 18) for supplying and discharging, by means of the valve device (46), a sample of the gaseous medium into and out of the infrared cuvette (10); wherein an output device (54) is connected to the signal-processing control and computing unit (52) on the output side, and
wherein the control and computing unit (52) is geared to carry out the sample analysis and in the process to measure the transmission of infrared radiation through the sample located in the infrared cuvette (10) at different wavelengths in the infrared spectrum and different pressures, to calculate therefrom the concentration of contaminants which influence the quality and to display this at least when predefinable quality parameters are exceeded;

**characterised in that**
the measuring apparatus (44) for monitoring the hydrogen quality can be connected to a hydrogen filling station or is integrated therein;
**in that** the infrared measuring device (42) is connected in the dispensing path, starting from the filling station, for online measurement of gas contaminants;
**in that** the medium dispensed is hydrogen;
**in that** the infrared cuvette (10) can be periodically filled with a sample of the hydrogen for measurement processes and emptied again as required by means of the valve device (46);
**in that** the sample analysis is carried out by means of the infrared measuring device (42) at very high pressures of >50 MPa;
**in that** the infrared measuring device (42) can be connected in the dispensing path in such a manner that samples can be taken in succession in a bypass to this dispensing path by means of the valve device (46) and can be examined for contaminants without the respective dispensing of hydrogen from the filling station to the connected consumer being interrupted; and
**in that** the infrared cuvette (10) is configured to be high-pressure-resistant and is provided with a pressure-resistant pipe section (12), wherein an infrared hollow conductor (14) is located inside the pipe section (12), which hollow conductor, configured as a galvanically gold-plated inner tube, is pushed into the pipe section (12) and fixed therein.

8. Measuring apparatus according to claim 7, **characterised in that** the pressure-resistant pipe section (12) comprises a flange part (22; 24) on at least one pipe end, which flange part has a pressure-resistant window (30) which transmits infrared radiation and/or a mirror (56) reflecting infrared radiation.

**Revendications**

1. Procédé de contrôle de la qualité de milieux gazeux pouvant être distribués par une station service, au moyen d'un dispositif (42) de mesure infrarouge, qui est monté dans le chemin de distribution du milieu gazeux respectif allant à un consommateur et qui mesure la transmission du rayonnement infrarouge à des longueurs d'ondes différentes du spectre infrarouge et des pressions, qui en calcule la concentration des impuretés influençant la qualité et, si au moins un paramètre de qualité pouvant être donné à l'avance est dépassé, met celui-ci à l'affichage, **caractérisé**

    **en ce que** l'on utilise le dispositif (42) de mesure infrarouge pour le contrôle de la qualité de l'hydrogène à une station service à hydrogène et en ce que l'on le monte, pour la mesure en ligne d'impuretés gazeuses, dans le chemin de distribution en partant de la station service ;

en ce que le milieu distribué est l'hydrogène ;

en ce que l'on effectue l'analyse d'échantillons au moyen du dispositif (42) de mesure infrarouge à des pressions très hautes de > 50 MPa ;

en ce que le dispositif (42) de mesure infrarouge est monté dans le chemin de distribution, de manière à prélever des échantillons successivement au moyen d'un dispositif (46) de soupape dans une dérivation à ce chemin de distribution, et à les contrôler en ce qui concerne les impuretés, sans qu'ainsi la distribution respective d'hydrogène de la station service au consommateur raccordé soit interrompue ; et

en ce que le dispositif (42) de mesure infrarouge comprend une cuvette (10) pour infrarouge de constitution résistante à la haute pression et ayant une pièce (12) tubulaire résistante à la pression, dans lequel, à l'intérieur de la pièce (12) tubulaire se trouve un guide d'onde (14) infrarouge, qui, constitué d'un tube intérieur doré galvaniquement, est inséré dans la pièce (12) tubulaire et y est fixé.

2. Procédé suivant la revendication 1, **caractérisé en ce que**, au moyen du dispositif (42) de mesure infrarouge, on détecte des impuretés, qui sont actives du point de vue infrarouge en raison d'un moment bipolaire.

3. Procédé suivant la revendication 1 ou 2, **caractérisé en ce que** le dispositif (42) de mesure infrarouge détermine, en utilisant la spectroscopie infrarouge à partir de la position de bande d'absorption d'un échantillon, le type de l'impureté et, à partir de l'atténuation du rayonnement infrarouge lorsqu'il traverse l'échantillon, détermine la concentration des impuretés respectives.

4. Procédé suivant l'une des revendications précédentes, **caractérisé en ce que**, pour la détermination de la concentration de l'impureté respective dans l'échantillon, on applique la loi de Lambert Beer.

5. Procédé suivant l'une des revendications précédentes, **caractérisé en ce que** l'on effectue l'analyse de l'échantillon au moyen du dispositif (42) infrarouge à des pressions très hautes de > 70 MPa.

6. Procédé suivant l'une des revendications précédentes, **caractérisé en ce qu'**une unité (52) de commande et de calcul du dispositif (42) de mesure infrarouge met en mémoire tampon des mesures de transmission de l'échantillon respectif à des densités différentes et les soustrait l'une de l'autre pour la détermination de la concentration et ce que, pour la détermination des densités de l'échantillon, on effectue une mesure de pression et, en cas de besoin supplémentairement, une mesure de température de l'échantillon.

7. Installation (44) de mesure pour le contrôle de la qualité de milieux gazeux pouvant être distribués par une station service, en particulier de l'hydrogène,

dans laquelle l'installation (44) de mesure comprend un dispositif (42) de mesure infrarouge ayant une cuvette (10) infrarouge, un émetteur (28) infrarouge et un détecteur (40) infrarouge, qui peut être monté dans le trajet de distribution du milieu gazeux respectif allant à un consommateur,

dans lequel l'installation (44) de mesure comprend en outre les composants suivants :

- un capteur (P) de pression ;
- une unité (52) de commande et de calcul ;
- un dispositif (46) de soupape ; et
- des raccordements (16, 18) pour l'arrivée et la sortie au moyen du dispositif (46) de soupape d'un échantillon du milieu gazeux, dans et hors de la cuvette (10) infrarouge, dans laquelle un appareil (54) d'édition est raccordé du côté de la sortie à l'unité (52) de commande et de calcul traitant un signal, et

dans laquelle l'unité (52) de commande et de calcul est agencée pour effectuer l'analyse de l'échantillon et pour mesurer la transmission du rayonnement infrarouge à travers l'échantillon se trouvant dans la cuvette (10) infrarouge à des longueurs d'ondes différentes du spectre infrarouge et des pressions pour en calculer la concentration d'impuretés influençant la qualité et pour, au moins lorsqu'un paramètre de qualité pouvant être donné à l'avance est dépassé, mettre celui-ci à l'affichage ;

**caractérisée**

en ce que le dispositif (44) de mesure, pour le contrôle de la qualité de l'hydrogène, peut être raccordé à une station service d'hydrogène ou y est intégré ;

en ce que le dispositif (42) de mesure infrarouge est, pour la mesure en ligne d'impuretés gazeuses, monté dans le chemin de distribution partant de la station service ;

en ce que le milieu distribué est l'hydrogène ;

en ce que la cuvette (10) infrarouge peut être emplie d'un échantillon de l'hydrogène et être vidée à nouveau, en cas de besoin périodiquement au moyen du dispositif (46) de soupape, pour des opérations de mesure ;

**en ce que** l'on effectue l'analyse de l'échantillon au moyen du dispositif (42) de mesure d'infrarouge à des pressions très hautes > 50 MPa ;
**en ce que** le dispositif (42) de mesure infrarouge peut être monté dans le chemin de distribution, de manière à pouvoir prélever des échantillons successivement dans une dérivation à ce chemin de distribution au moyen du dispositif (46) de soupape, et à pouvoir les contrôler, en ce qui concerne des impuretés,
sans interrompre pour autant la distribution respective d'hydrogène de la station service au consommateur raccordé ; et
**en ce que** la cuvette (10) infrarouge est de constitution résistante aux très hautes pressions et est pourvue d'une pièce (12) tubulaire résistante à la pression, dans laquelle, à l'intérieur de la pièce (12) tubulaire, se trouve un guide d'onde (14) infrarouge, qui, constitué sous la forme d'un tube intérieur doré galvaniquement, est introduit dans la pièce (12) tubulaire et y est fixé.

8.  Installation de mesure suivant la revendication 7, **caractérisée en ce que**
    la pièce (12) tubulaire résistante à la pression comprend, à au moins une extrémité du tube, une partie (22 ; 24) de bride, qui a une fenêtre (30) résistante à la pression et perméable au rayonnement infrarouge et/ou un miroir (56) réfléchissant le rayonnement infrarouge.

Fig.1

Fig.2

Fig.3

Fig.4

Fig.5

Fig.6

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013139462 A1
- US 20150131093 A1
- GB 2466181 A
- US 20110005302 A1
- US 5223716 A
- US 7446317 B2
- DE 19900129 A1
- WO 2009155459 A2